(19) Europäisches Patentamt
European Patent Office
Office européen des brevets

(11) Veröffentlichungsnummer: **0 001 654**
**B1**

(12) # EUROPÄISCHE PATENTSCHRIFT

(45) Veröffentlichungstag der Patentschrift: **22.07.81**

(51) Int. Cl.³: **C 07 D 233/60**, A 01 N 43/50, A 61 K 31/415, C 07 D 401/12

(21) Anmeldenummer: **78200225.7**

(22) Anmeldetag: **04.10.78**

(54) **Imidazolyläthyl-oxyalkoxy-Derivate und ihre Thio-Analoge, pharmazeutische Zubereitungen mit diesen Stoffen und Verfahren zu ihrer Herstellung.**

(30) Priorität: **13.10.77 CH 12528/77**

(43) Veröffentlichungstag der Anmeldung: **02.05.79 Patentblatt 79/9**

(45) Bekanntmachung des Hinweises auf die Patenterteilung: **22.07.81 Patentblatt 81/29**

(84) Benannte Vertragsstaaten: **BE CH DE FR GB NL SE**

(56) Entgegenhaltungen:
**DE-A-127 186**
**DE-A-1 595 994**
**DE-A-1 720 042**
**DE-A-1 940 388**
**DE-A-2 361 391**
**DE-A-2 619 381**

(73) Patentinhaber: **SIEGFRIED AKTIENGESELLSCHAFT, CH-4800 Zofingen (CH)**

(72) Erfinder: **Zirngibl, Ludwig, Dr., Eisengrubenweg 16, CH-4800 Zofingen (CH)**
Erfinder: **Molnar, Istvan, Dr., Eschenweg 10, CH-4800 Zofingen (CH)**
Erfinder: **Thiele, Kurt, Dr., Rebbergstrasse 47, CH-4800 Zofingen (CH)**
Erfinder: **Fischer, Johanna, Dr., Mattenstrasse 10, CH-6260 Reiden (CH)**

(74) Vertreter: **Ritscher, Thomas, Dr. et al, RITSCHER & SEIFERT Auf der Mauer 4, CH-8001 Zürich (CH)**

**EP 0 001 654 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Imidazolyläthyl-oxyalkoxy-Derivate und ihre Thio-Analoge, pharmazeutische Zubereitungen mit diesen Stoffen und Verfahren zu ihrer Herstellung

Die Erfindung betrifft neue, gegebenenfalls als Säureadditionssalze vorliegende Imidazolyläthyl-oxyalkoxy-Derivate einschließlich ihrer Thio-Analoga, die beide dank ihrer antimykotischen und antibakteriellen Wirkung für die Verwendung in der Human- und Veterinärmedizin sowie für den Pflanzenschutz geeignet sind. Die Erfindung betrifft ferner pharmazeutische Zubereitungen, welche die neuen Wirkstoffe enthalten, und Verfahren zur Herstellung Wirkstoffe.

Verschiedene Imidazolderivate und Salze hiervon mit fungizider bzw. antimykotischer Wirkung sind bekannt, z. B. die 1-($\beta$-Aryl)-äthylimidazole (DE-OS 1 940 388) sowie die 1-Aryl-2-(1-imidazolyl)-alkyläther bzw. -äthylthioäther (DE-OS 2 619 381). Eine aus diesem Stand der Technik ableitbare allgemeine Struktur bekannter Imidazolderivate entspricht daher der Formel (10)

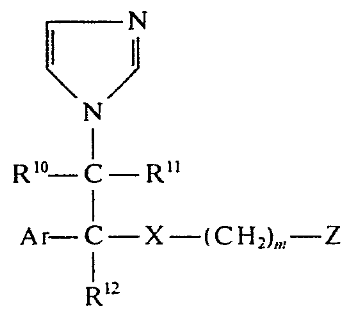

(10)

und die Verbindungen (10) bzw. ihre Salze sind von erheblichem pharmakologischem Interesse für die Bekämpfung von Mykosen insbesondere dann, wenn die allgemeinen Symbole der Formel (10) folgende Bedeutung haben: $R^{10}$ bis $R^{12}$ sind Wasserstoffatome oder Niederalkylreste, X ist Sauerstoff oder Schwefel, Ar ist ein gegebenenfalls substituierter Arylrest, Z ist eine aromatische oder heteroaromatische Gruppe und m bedeutet Null, 1 oder 2. Dabei kann der 1-Imidazolylrest gegebenenfalls mindestens einen zusätzlichen Substituenten, z. B. Niederalkyl, tragen.

Für chemotherapeutische Zwecke, insbesondere bei oraler sowie auch bei rektaler oder vaginaler Applikation von Antimycotica zur Behandlung systemischer Mykosen sind — abgesehen von der antimykotischen Wirksamkeit — insbesondere die orale und/oder die Löslichkeit des Wirkstoffes von vorrangiger Bedeutung. Dasselbe gilt mutatis mutandis zugleich auch für die Verwendung von Fungiziden für den Pflanzenschutz. Es wurde gefunden, daß bedeutsame Vorteile in dieser Hinsicht praktisch ohne Beeinträchtigung oder sogar mit Verbesserungen der antimykotischen Wirkung erzielt werden können, wenn die Struktur der Formel (10) erfindungsgemäß durch eine in die Kette zwischen der Alkylenbrücke $-(CH_2)_m$ und die Gruppe Z zusätzlich eingeführte Sauerstoff- oder Schwefelbrücke modifiziert wird, d. h. die Kette der Struktur (20)

$$Ar-\overset{|}{\underset{|}{C}}-X-(CH_2)_m-Z \qquad (20)$$

wahlweise wie folgt ausgebildet ist:

$$Ar-\overset{|}{\underset{|}{C}}-O-(CH_2)_n-O-R \qquad (21)$$

$$Ar-\overset{|}{\underset{|}{C}}-O-(CH_2)_n-S-R \qquad (22)$$

$$Ar-\overset{|}{\underset{|}{C}}-S-(CH_2)_n-O-R \qquad (23)$$

$$Ar-\overset{|}{\underset{|}{C}}-S-(CH_2)_n-S-R \qquad (24)$$

worin R Wasserstoff, eine Niederalkyl- oder eine gegebenenfalls substituierte Phenylgruppe ist und n 1, 2 oder 3 bedeutet.

Dabei ist zu bemerken, daß bei einigen der aus der obengenannten DE-OS 2 619 381 bekannten Verbindungen der Formel (10) die oben als Z bezeichnete Gruppe eine Thienylgruppe sein kann. Dies ließ aber keinesfalls die Erwartung zu, daß Verbindungen (10) mit den modifizierten Kettenstrukturen (21) bis (24) vergleichbare oder sogar verbesserte pharmakologische Eigenschaften haben würden, da der Schwefel eines heteroaromatischen Ringes allgemein nicht mit dem Schwefel in acyclischer Bindung gleichgesetzt werden kann.

Daß den Kettenstrukturen (21) bis (24) eine erhebliche Bedeutung zukommt, ergibt sich ferner aus dem überraschenden Befund, daß die Gruppe Z in Formel (10), die entsprechend den genannten Offenlegungsschriften eine aromatische oder heteroaromatische Gruppe sein muß, nicht nur durch eine aliphatische Gruppe, sondern sogar durch das Wasserstoffatom ersetzt werden kann, ohne daß dies die antimykotische Wirkung aufhebt.

Die Erfindung betrifft neue Verbindungen mit dieser charakteristischen Kette sowie die entsprechenden Additionssalze mit pharmakologisch zulässigen Säuren. Die Erfindung betrifft ferner Verfahren zur Herstellung der neuen Verbindungen sowie antimykotische Mittel für die orale Verabreichung der neuen Verbindungen in der Human- oder Veterinärmedizin.

Die neuen Verbindungen gemäß der Erfindung sind gekennzeichnet durch die Formel (1)

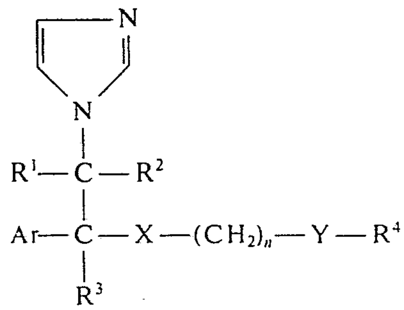

(1)

in welcher

| | |
|---|---|
| $R^1$, $R^2$ und $R^3$ | gleich oder verschieden und Wasserstoffatome oder Niederalkylgruppen sind, |
| $R^4$ | das Wasserstoffatom, eine Niederalkylgruppe, eine $C_4$—$C_8$-Cycloalkyl-, Benzyl- oder Phenylgruppe, die gegebenenfalls mindestens einen Substituenten trägt, der ein Halogenatom oder eine Niederalkyl-, Niederalkoxy-, Niederalkylthio-, Phenyl-, Cyano-, Nitro- oder Aminogruppe ist, oder eine gegebenenfalls halogensubstituierte Pyrodylgruppe bedeutet, |
| Ar | eine Phenylgruppe bedeutet, die gegebenenfalls mindestens einen Substituenten trägt, der ein Halogenatom oder eine Niederalkyl-, Niederalkoxy- oder $C_4$—$C_8$-Cycloalkylgruppe oder eine gegebenenfalls mit einem der für $R^4$ aufgezählten Substituenten substituierte Benzylgruppe ist, |
| X und Y | unabhängig voneinander Sauerstoff- oder Schwefelatome bedeuten und |
| n | eine Zahl von 1 bis 5 darstellt. |

Die Erfindung umfaßt auch die pharmakologisch zulässigen Säureadditionssalze von Verbindungen der Formel (1).

Die neuen Verbindungen (1) haben wie die bekannten Verbindungen (10) mindestens ein asymmetrisches C-Atom (das $\beta$-C-Atom) und die Erfindung umfaßt sowohl die Racemate als auch die Stereoisomeren.

Wenn bei den neuen Verbindungen (1) $R^1$ ungleich $R^2$ ist, so ist auch das $\alpha$-C-Atom asymmetrisch, d. h. die Verbindungen (1) können zwei asymmetrische C-Atome enthalten und auch in diesem Fall soll die Erfindung die jeweiligen Racemate und Stereoisomeren umfassen. Überraschenderweise zeigen Verbindungen (1) mit asymmetrischem $\alpha$-C-Atom eine nochmals erheblich günstigere orale Toxizität an Mäusen (siehe Beispiel 25), d. h. einen höheren $LD_{50}$-Wert.

Beispiele für geeignete anorganische oder organische Säuren zur Bildung der Additionssalze sind in den genannten Offenlegungsschriften angegeben. Die Nitrate werden für viele Zwecke bevorzugt. Weitere Beispiele sind Halogenwasserstoffsäuren, d. h. Chlorwasserstoffsäure, Bromwasserstoffsäure oder Jodwasserstoffsäure, Schwefelsäure, Thiocyansäure, Phosphorsäuren, Essigsäure, Propionsäure, Glycolsäure, Milchsäure, Brenztraubensäure, Oxalsäure, Malonsäure, Bernsteinsäure, Maleinsäure, Fumarsäure, Äpfelsäure, Weinsäure, Citronensäure, Benzoesäure, Zimtsäure, Mandelsäure, Methansulfonsäure, Äthansulfonsäure, Hydroxyäthansulfonsäure, p-Toluolsulfonsäure, Salicylsäure, p-Aminosalicylsäure, 2-Phenoxybenzoesäure oder 2-Acetoxybenzoesäure.

In der obigen Definition zur Formel (1) bedeutet »Niederalkyl« bzw. »Niederalkoxy« und »Niederalkylthio« Gruppen mit 1—6 C-Atomen in gerader oder verzweigter Kette. Methyl ist eine bevorzugte Gruppe für Niederalkyl, Niederalkoxy und Niederalkylthio. Halogen bedeutet Fluor, Chlor, Brom und Jod. Chlor und Brom werden meist bevorzugt.

Bei einer Gruppe bevorzugter Verbindungen der Formel (1) bedeutet $R^1$ Wasserstoff oder Methyl, während $R^2$ und $R^3$ Wasserstoffatome sind. Ferner werden meist Verbindungen (1) bevorzugt, bei welchen n eine Zahl von 1 bis 4, insbesondere 1 bis 3 ist. Viele Verbindungen, bei welchen n 2 oder 3 ist, sind besonders vorteilhaft. Bevorzugte Beispiele von $R^4$ sind Methyl als Niederalkyl, gegebenenfalls monohalogensubstituierte, insbesondere chlorsubstituierte Benzyl- oder Cyclohexylgruppen, die gegebenenfalls mono- oder disubstituierte Phenylgruppe, deren Substituenten ein oder zwei Halogenatome, vorzugsweise Chlor oder/und Brom, eine Niederalkyl-, Niederalkoxy- oder Niederalkylthiogruppe, gegebenenfalls zusammen mit einem Halogenatom als zweitem Substituent, oder eine Phenylgruppe sind. Spezielle Beispiele substituierter Phenylgruppen sind 2- oder 4-Halogenphenyl (mit Cl oder Br), 2,4- oder 3,4-Dihalogenphenyl (mit Cl oder/und Br), 2- oder 4-Methylphenyl, 2- oder 4-Methoxyphenyl, 4-Methylthiophenyl, 4-Phenyl-phenyl, 3-Methyl-4-bromphenyl usw. 4-Chlorbenzyl und 5-Chlorpyrid-2-yl sind weitere geeignete Beispiele.

Bevorzugte Beispiele für Ar sind Phenylgruppen, die durch ein oder zwei Halogenatome, durch eine gegebenenfalls bzw. vorzugsweise monohalogensubstituierte Benzyl- oder Cyclohexylgruppe substituiert sind. Ein bevorzugtes spezielles Beispiel für Ar ist die 2,4-Dichlorphenylgruppe.

Bei einer weiteren bevorzugten Gruppe von Verbindungen (1) ist Y Sauerstoff, wenn $R^4$ Wasserstoff oder Niederalkyl ist. Ebenfalls bevorzugt werden Verbindungen (1), bei welchen $R^4$ den 4-Chlorphenylrest und (a) X Sauerstoff, Y Schwefel und n 1 bedeutet oder (b) beide X und Y Schwefel sind und n 2 ist.

Spezielle bevorzugte Verbindungen (1) werden in den Beispielen weiter unten genannt.

Die Verbindungen (1) können nach an sich bekannten Verfahren hergestellt werden, z. B. nach einem der folgenden Schemata (I) bis (IV):

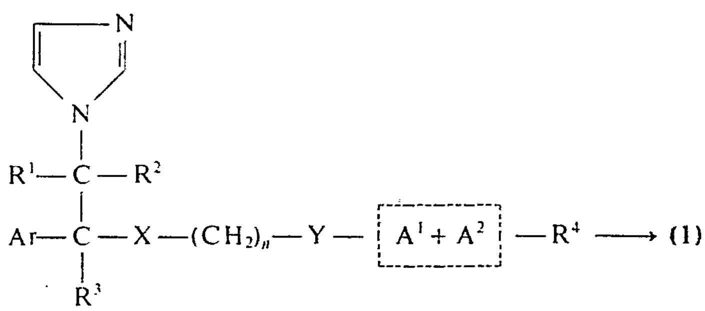

(I)

(11) (21)

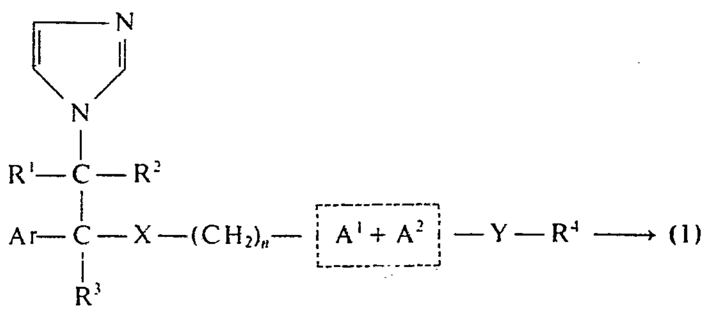

(II)

(12) (22)

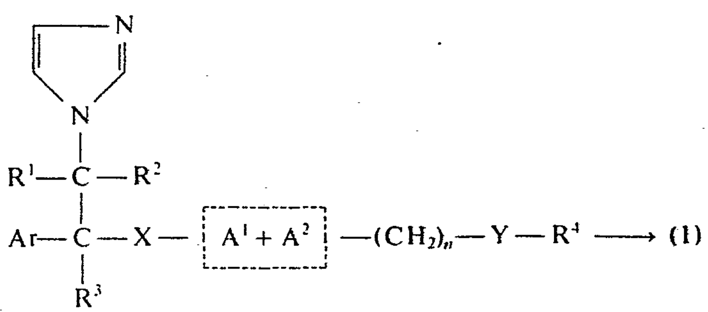

(III)

(13) (23)

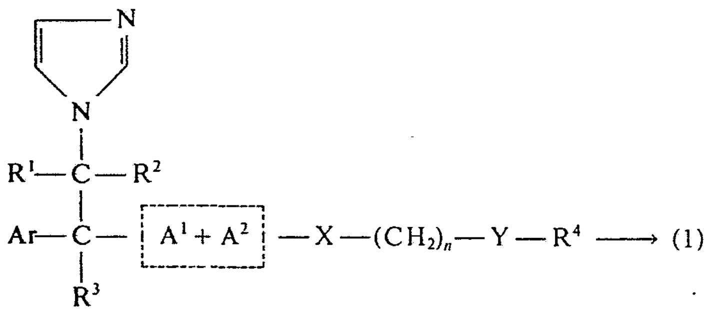

(IV)

(14) (24)

wobei $A^1$ und $A^2$ entsprechende Abgangsgruppen bedeuten, die unter den Reaktionsbedingungen unter Bildung der Zielprodukte (1) abgespalten werden. Wenn $A^1$ bzw. $A^2$ z. B. Wasserstoff bedeutet, ist es meist zweckmäßig, die entsprechende Hydroxyl- bzw. Thiolgruppe vor der Kondensation mit den entsprechenden anderen Gruppen $A^1$, $A^2$, die z. B. Halogen-, insbesondere Chloratome oder auch Methylsulfonyl- oder p-Methylbenzolsulfonylgruppen sind, zunächst in ein Metallalkoholat bzw. -thiolat umzuwandeln, das als Metall ein Alkalimetall enthält.

Geeignete Kondensationsmethoden, Kondensationsmittel und Reaktionsmedien für Synthesen entsprechen den Schemata (I) bis (IV) gehören an sich ebenso zum Stand der Technik, wie die Herstellung der jeweils erforderlichen Ausgangsverbindungen, so daß sich eine eingehendere Erläuterung erübrigt.

Beispiel 1

1-β-(Methoxy-methoxy-2,4-dichlorphenäthyl)-imidazol-nitrat

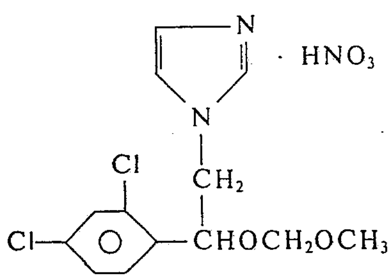

In einem Dreihalskolben mit Innenthermometer, Rückflußkühler und Magnetrührer löst man 7,71 g α-(2,4-Dichlorphenyl)-imidazol-1-äthanol (Herstellung gemäß Godefroi et al, J. med. Chem. 12, 784, 1969) in 20 ml Hexamethylphosphorsäuretriamid (»HEMPA«) auf und fügt 1,68 g einer 50%igen Dispersion von Natriumhydrid in Mineralöl hinzu. Die Temperatur steigt unter starkem Schäumen auf 40° C. Dann wird 60 min bei Raumtemperatur gerührt und abschließend 60 min auf 40—60° C erwärmt. Dann tropft man bei einer Kolbeninnentemperatur von 5—10° C 2,98 g Chlordimethyläther zu, läßt auf Raumtemperatur kommen und 15 Std. stehen.

Die Reaktionsmischung wird auf 450 ml Wasser gegossen. Man extrahiert im Scheidetrichter mit dreimal 150 ml Essigester. Die vereinigten organischen Phasen werden mit Natriumsulfat getrocknet, filtriert und das Filtrat im Vakuum eingedampft. Der Rückstand beträgt 11,2 g dunkles Öl, welches man mittels Dichlormethan an Kieselgel (Präparat für Trockensäulenchromatographie der Firma Woelm) chromatographiert. Die Eluate mit reinem Produkt werden vereinigt und eingedampft. Der Verdampfungsrückstand wird mit Essigester/Äther gelöst. Man fällt das Produkt mit 65%iger Salpetersäure und erhält 3,6 g Zielprodukt in Form von nahezu farblosen Kristallen. Nach dem Umkristallisieren aus Wasser/Methanol (9 : 1) verbleiben 2,91 g reines Zielprodukt (27% der theor. Ausbeute) in Form von farblosen Kristallen, F 122—123° C.

Analyse (%)
berechnet für $C_{13}H_{14}Cl_2N_2O_2 \cdot HNO_3$:
42,88 C, 4,15 H, 11,54 N
gefunden:
42,44 C, 4,11 H, 11,34 N

Beispiele 2—20

Die folgenden Verbindungen der Formel (1) sind in Analogie zu dem in Beispiel 1 beschriebenen Verfahren erhältlich:

2. 1-β-(Phenoxymethoxy-2,4-dichlorphenäthyl)-imidazol-nitrat, F 138—144° C.
3. 1-β-(2-Chlorphenoxymethoxy-2,4-dichlorphenäthyl)-imidazol-nitrat, F 152—153° C.
4. 1-β-(4-Chlorphenoxymethoxy-2,4-dichlorphenäthyl)-imidazol-nitrat, F 144—145° C.
5. 1-β-(2,4-Dichlorphenoxymethoxy-2,4-dichlorphenäthyl)-imidazol-nitrat, F 154—157° C.
6. 1-β-(4-Methylphenoxymethoxy-2,4-dichlorphenäthyl)-imidazol-nitrat, F 158—159° C.
7. 1-β-(4-Methoxyphenoxymethoxy-2,4-dichlorphenyl)-imidazol-nitrat, F 130—132° C.
8. 1-β-(4-Methylthiophenoxymethoxy-2,4-dichlorphenäthyl)-imidazol-nitrat, F 142—143° C.
9. 1-β-(4-Phenylphenoxymethoxy-2,4-dichlorphenäthyl)-imidazol-nitrat, F 172—173° C.
10. 1-β-(Methylthiomethoxy-2,4-dichlorphenäthyl)-imidazolnitrat, F 144—145° C.
11. 1-β-(Phenylthiomethoxy-2,4-dichlorphenäthyl)-imidazol-nitrat, F 142—143° C.
12. 1-β-(4-Bromphenylthiomethoxy-2,4-dichlorphenäthyl)-imidazol-nitrat, F 155—157° C.
13. 1-β-(4-Chlorphenylthiomethoxy-2,4-dichlorphenäthyl)-imidazol-nitrat, F 140—142° C.
14. 1-β-(4-Methylphenylthiomethoxy-2,4-dichlorphenäthyl)-imidazol-nitrat, F 144—145° C.
15. 1-β-(3,4-Dichlorphenylthiomethoxy-2,4-dichlorphenäthyl)-imidazol-nitrat, F 112—113° C.
16. 1-β-(4-Brom-3-methylphenylthiomethoxy-2,4-dichlorphenäthyl)-imidazol-nitrat, F 127—129° C.
17. 1-β-(4-Cyclohexylphenäthyl-β'-hydroxyäthoxy)-imidazol-hydrochlorid, F 163—164° C.
18. 1-β-(2,4-Dichlorphenäthyl-β'-hydroxyäthylthio)-imidazol-nitrat, F 90—92° C.
19. 1-[β-Methylthiomethoxy-4(4'-chlorbenzyl)-phenäthyl]-imidazol-nitrat, F 145—147° C.
20. 1-β-(3-Phenoxypropyloxy-2,4-dichlorphenäthyl)-imidazol-methansulfonat, F 90—92° C.

Beispiel 21

1-β-(4-Chlorphenylthioäthylthio-2,4-dichlorphenäthyl)-imidazol

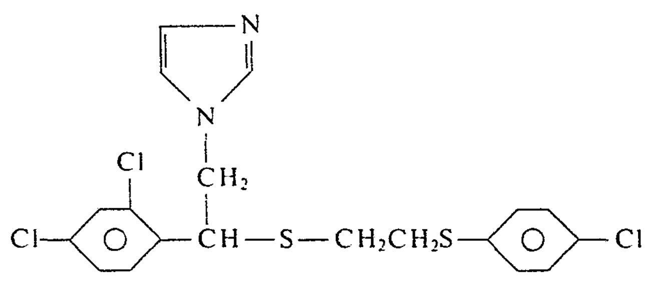

Die Synthese erfolgte nach dem oben angegebenen Schema (IV) mit $A^1$ = Cl und $A^2$ = H. Die Verbindung (24) von Schema (IV) ist hier also das 2-(4-Chlorphenylthio)-äthylmercaptan, das wie folgt erhältlich ist: Eine Mischung aus 14,5 g p-Chlorphenol, 6,61 g Äthylensulfid, 1,25 ml Triäthylamin und 50 ml Petroläther (Siedebereich 35—70° C) wird 7 Std. unter Rückfluß erhitzt. Die Sumpftemperatur beträgt dabei etwa 50° C. Man läßt über Nacht stehen. Die Reaktionsmischung wird im Vakuum eingedampft. Der Rückstand, der in einer Menge von 20,36 g in Form eines öligen Produktes anfällt, wird destilliert und ergibt 14,3 g reines 2-(4-Chlorphenylthio)-äthylmercaptan in einer Ausbeute von 70%, Kp 110—112° C/0,5 mm, n 1,6238.

Analyse (%)
berechnet für $C_8H_9ClS_2$:
46,93 C, 4,43 H
gefunden:
47,20 C, 4,56 H.

Eine Mischung aus 10,23 g des so erhaltenen Mercaptans mit 19,00 g 1-(2,4-Dichlorphenyl)-2-(1-imidazolyl)-äthylchlorid in Form des Hydrochlorid-Semizinkchloridsalzes, 16,6 g gepulvertes wasserfreies Kaliumcarbonat und 300 ml Methylisobutylketon wird 12 Std. zum Rückfluß erhitzt. Die Reaktionsmischung wird heiß filtriert und das Filtrat im Vakuum eingedampft. Es bleiben 24,5 g Rückstand in Form eines hellbraunen Öls zurück, das mit Methylenchlorid an Kieselgel (Präparat zur Trockensäulenchromatographie, Firma Woelm) chromatographiert wird. Die reinen Fraktionen werden vereinigt und eingedampft. Man erhält 12,3 g (55% Ausbeute) des Zielproduktes in Form eines hellen Öls.

Analyse (%)
berechnet für $C_{19}H_{17}Cl_3N_2S_2$:
51,41 C, 3,86 H, 6,31 N
gefunden:
51,27 C, 3,90 H, 6,18 N.

Beispiele 22—28

Nach dem Verfahren von Beispiel 1 wurden mit entsprechend modifizierten Ausgangsstoffen folgende Verbindungen hergestellt:

22. 1-[β-(Cyclohexylthiomethoxy-2,4-dichlorphenäthyl)]-imidazol-nitrat, F 144—145° C.
23. 1-β-(p-Chlorbenzylthiomethoxy-2,4-dichlorphenäthyl)-imidazol-nitrat, F 139—140° C.
24. 1-β-(2-Methoxyphenoxymethyloxy-2,4-dichlorphenäthyl)-imidazol-nitrat, F 125—126° C.
25. 1-[β-(p-Chlorphenylthiomethoxy)-2,4-dichlorphenäthyl-α-methyl]-imidazol-nitrat, F 115—126° C.
26. 1-β[(5'-Chlorpyridyl-2'-thio-2-methoxy)-2,4-dichlorphenäthyl)]-imidazol-nitrat, F 159—161° C.
27. 1-β-(Phenoxyäthoxy-2,4-dichlorphenäthyl)-imidazol-nitrat, F 111—112° C.
28. 1-[β-(p-Chlorphenoxyäthoxy)-2,4-dichlorphenäthyl]-imidazol-nitrat, F 129,5—132,5° C.

Beispiel 29

Nach dem in Beispiel 21 beschriebenen Verfahren wird aus Äthylensulfid und 5-Chlorpyridin-2-mercaptan in Tetrahydrofuran das 2-(5'-Chlorpyridyl-2'-thio)-äthylmercaptan hergestellt, welches wie in Beispiel 2 weiter zum Zielprodukt von Beispiel 29, dem 1-{β-[(5'-Chlorpyridyl-2'-thio-2-äthylthio)-2,4-dichlorphenäthyl]}-imidazol umgesetzt wird. Die freie Base wird in Form eines Sirups erhalten.

Analyse (%)
berechnet für $C_{18}H_{16}Cl_3N_3S_2$:
48,60 C, 3,63 H, 9,45 N, 14,42 S, 23,91 Cl
gefunden:
48,76 C, 3,75 H, 9,10 N, 14,20 S, 24,30 Cl

Beispiele 30—31

Nach dem in Beispiel 1 beschriebenen Verfahren werden mit entsprechend abgeänderten Ausgangsstoffen folgende Verbindungen hergestellt:

30. 1-{β-[3-(p-Chlorphenoxy)-propoxy-2,4-dichlorphenäthyl]}-imidazol-nitrat, F 118—119° C.
31. 1-{β-[3-(p-Chlorphenylthio)-propoxy]-2,4-dichlorphenäthyl}-imidazol-nitrat, F 108—110° C.

Beispiel 32

3-Phenoxypropylbromid wird in 95%igem Äthanol 4 Std. unter Rückfluß mit Thioharnstoff zum 3-Phenoxypropylmercaptan, Kp 121—123° C/9 mbar, umgesetzt und dieses nach dem Verfahren von Beispiel 21 zum Zielprodukt von Beispiel 32, dem 1-[β-(Phenoxypropylthio)-2,4-dichlorphenäthyl]-imidazol umgesetzt.

Diese Base wird als dickes Öl erhalten und durch Säulenchromatographie an Kieselgel mit $CH_2Cl_2/CHCl_3$ gereinigt.

Analyse (%)
berechnet für $C_{20}H_{20}Cl_2N_2OS$:
58,97 C, 4,95 H, 6,88 N, 7,87 S, 17,41 Cl
gefunden:
59,01 C, 5,13 H, 6,52 N, 7,50 S, 17,63 Cl.

Beispiel 33

Nach dem Verfahren von Beispiel 32 wird 3-(4-Chlorphenoxy)-propylmercaptan, Kp 83—93° C/0,006 mbar, hergestellt und nach dem Verfahren von Beispiel 21 zum Zielprodukt von Beispiel 33, dem 1{β-[3-(p-Chlorphenoxy)-propylthio-2,4-dichlorphenäthyl]}-imidazol, umgesetzt, das wie in Beispiel 32 gereinigt wurde und folgende Analysedaten lieferte:

Analyse (%)
berechnet für $C_{19}H_{17}Cl_3N_2S_2$:
51,41 C, 3,86 H, 6,31 N
gefunden:
51,27 C, 3,90 H, 6,18 N.

Beispiele 22—28

Nach dem Verfahren von Beispiel 1 wurden mit entsprechend modifizierten Ausgangsstoffen folgende Verbindungen hergestellt:

22. 1-[β-(Cyclohexylthiomethoxy-2,4-dichlorphenäthyl)]-imidazol-nitrat, F 144—145° C.
23. 1-β-(p-Chlorbenzylthiomethoxy-2,4-dichlorphenäthyl)-imidazol-nitrat, F 139—140° C.
24. 1-β-(2-Methoxyphenoxymethyloxy-2,4-dichlorphenäthyl)-imidazol-nitrat, F 125—126° C.
25. 1-[β-(p-Chlorphenylthiomethoxy)-2,4-dichlorphenäthyl-α-methyl]-imidazol-nitrat, F 115—126° C.
26. 1-β[(5'-Chlorpyridyl-2'-thio-2-methoxy)-2,4-dichlorphenäthyl)]-imidazol-nitrat, F 159—161° C.
27. 1-β-(Phenoxyäthoxy-2,4-dichlorphenäthyl)-imidazol-nitrat, F 111—112° C.
28. 1-[β-(p-Chlorphenoxyäthoxy)-2,4-dichlorphenäthyl]-imidazol-nitrat, F 129,5—132,5° C.

Beispiel 29

Nach dem in Beispiel 21 beschriebenen Verfahren wird aus Äthylensulfid und 5-Chlorpyridin-2-mercaptan in Tetrahydrofuran das 2-(5'-Chlorpyridyl-2'-thio-äthylmercaptan hergestellt, welches wie in Beispiel 2 weiter zum Zielprodukt von Beispiel 29, dem 1-{β-[(5'-Chlorpyridyl-2'-thio-2-äthylthio)-2,4-dichlorphenäthyl]}-imidazol umgesetzt wird. Die freie Base wird in Form eines Sirups erhalten.

Analyse (%)
berechnet für $C_{18}H_{16}Cl_3N_3S_2$:
48,60 C, 3,63 H, 9,45 N, 14,42 S, 23,91 Cl
gefunden:
48,76 C, 3,75 H, 9,10 N, 14,20 S, 24,30 Cl

Beispiele 30—31

Nach dem in Beispiel 1 beschriebenen Verfahren werden mit entsprechend abgeänderten Ausgangsstoffen folgende Verbindungen hergestellt:

30. 1-{β-[3-(p-Chlorphenoxy)-propoxy-2,4-dichlorphenäthyl]}-imidazol-nitrat, F 118—119° C.
31. 1-{β-[3-(p-Chlorphenylthio)-propoxy]-2,4-dichlorphenäthyl}-imidazol-nitrat, F 108—110° C.

Beispiel 32

3-Phenoxypropylbromid wird in 95%igem Äthanol 4 Std. unter Rückfluß mit Thioharnstoff zum 3-Phenoxypropylmercaptan, Kp 121—123° C/9 mbar, umgesetzt und dieses nach dem Verfahren von Beispiel 21 zum Zielprodukt von Beispiel 32, dem 1-[β-(Phenoxypropylthio)-2,4-dichlorphenäthyl]-imidazol umgesetzt.
Diese Base wird als dickes Öl erhalten und durch Säulenchromatographie an Kieselgel mit $CH_2Cl_2/CHCl_3$ gereinigt.

Analyse (%)
berechnet für $C_{20}H_{20}Cl_2N_2OS$:
58,97 C, 4,95 H, 6,88 N, 7,87 S, 17,41 Cl
gefunden:
59,01 C, 5,13 H, 6,52 N, 7,50 S, 17,63 Cl.

Beispiel 33

Nach dem Verfahren von Beispiel 32 wird 3-(4-Chlorphenoxy)-propylmercaptan, Kp 83—93° C/0,006 mbar, hergestellt und nach dem Verfahren von Beispiel 21 zum Zielprodukt von Beispiel 33, dem 1{β-[3-(p-Chlorphenoxy)-propylthio-2,4-dichlorphenäthyl]}-imidazol, umgesetzt, das wie in Beispiel 32 gereinigt wurde und folgende Analysedaten lieferte:

Analyse (%)
berechnet für $C_{20}H_{19}Cl_3N_2OS$:
54,37 C, 4,33 H, 6,34 N
gefunden:
54,44 C, 4,72 H, 6,02 N

Beispiel 34

Nach dem Verfahren von Beispiel 32 wird aus 3-(4-Chlorphenylthio)-propylchlorid (Kp 116—120°C/0,4 mbar) das 3-(4-Chlorphenylthio)-propylmercaptan (Kp 120—125°C/0,2 mbar) hergestellt und dieses dann nach dem Verfahren von Beispiel 21 zum Zielprodukt von Beispiel 34 umgesetzt, dem 1-{β-[3-(p-Chlorphenylthiopropylthio)]-2,4-dichlorphenäthyl}-imidazol-nitrat, Fp 79—83°C. Die Reinigung des Produktes erfolgte wie in Beispiel 32.

Beispiele 35—36

Nach dem Verfahren von Beispiel 1 wurde aus 4-(4-Chlorphenoxy)-butylbromid (Kp 114—120°C/0,6 mbar) das Zielprodukt von Beispiel 35, nämlich das 1-{β-[4-(p-Chlorphenoxy)-butoxy]-2,4-dichlorphenäthyl}-imidazol-nitrat, F 110—113°C, erhalten.

Das als Ausgangsstoff verwendete Material wurde durch 6 Std. Erhitzen von 1,4-Dibrombutan, 4-Chlorphenol und $K_2CO_3$ in Dimethylformamid erhalten. In analoger Weise wurde aus 4-(4-Chlorphenylthio)-butylbromid (Kp 132—135°C/0,05 mbar) das Zielprodukt von Beispiel 36, nämlich 1-{β-[4-(p-Chlorphenylthio)-butoxy]-2,4-dichlorphenäthyl}-imidazol-nitrat, F 105—108°C, erhalten.

Die nach den Beispielen 1—21 erhaltenen Verbindungen (1) bzw. Säureadditionssalze wurden zur Bestimmung der minimalen Hemmkonzentration (in µg/ml) für verschiedene Bakterien und Kleinpilze nach der bekannten Gradientenplattenmethode mit Gradienten von Null bis 100 µg/ml als Lösung in 10%igem Dimethylformamid getestet. Die Testablesung erfolgte nach drei Tagen. Die Ergebnisse sind in der folgenden Tabelle I gemeinsam mit entsprechenden Daten der Vergleichssubstanz Econazol (Toxizitätswert gemäß Thienpont et al, Arzneimittelforschung, 25, 1975, 224) zusammengestellt; Econazol ist 1-{β-[(4-Chlor-phenyl)methoxy]-2,4-dichlorphenäthyl}-imidazol.

Tabelle I

| Beisp. No. | Toxizität $LD_{50}$ per os (mg/kg) (Maus) | Minimale Hemmkonzentration (µg/ml) | | | |
|---|---|---|---|---|---|
| | | Bakterien St | Str | Pilze Tri | Asp |
| 1 | 540 | | | <10 | <10 |
| 2 | >3000 | 10 | 25 | <10 | <10 |
| 3 | >3000 | <10 | 15 | <10 | <10 |
| 4 | ca. 2750 | <10 | <10 | <10 | <10 |
| 5 | ⩾3000 | <10 | <10 | <10 | <10 |
| 6 | >3000 | <10 | 15 | <10 | <10 |
| 7 | 2600 | 10 | 30 | <10 | <10 |
| 8 | ca. 2100 | 10 | | <10 | <10 |
| 9 | ca. 3500 | | | | |
| 10 | ca. 950 | 20 | 30 | <10 | <10 |
| 11 | >3000 | <10 | 20 | <10 | <10 |
| 12 | 12 500 | <10 | 10 | <10 | <10 |
| 13 | 3850 | 10 | 10 | <10 | <10 |

Fortsetzung

| Beisp. No. | Toxizität $LD_{50}$ per os (mg/kg) (Maus) | Minimale Hemmkonzentration (µg/ml) Bakterien St | Str | Pilze Tri | Asp |
|---|---|---|---|---|---|
| 14 | ⩾3000 | <10 | <10 | <10 | <10 |
| 15 | ~7500 | <10 | <10 | <10 | 25 r |
| 16 | ⩾3000 | <10 | 10 | <10 | <10 |
| 17 | ⩾1000 | 50 | | 30 | 60 |
| 18 | 1300 | 50 | 40 | 30 | |
| 19 | 970 | 10 | 25 | <10 | 10 |
| 20 | ~2400 | <10 | <10 | <10 | <10 |
| 21 | >3000 | <10 | | <10 | <10 |
| 22 | >3000 | <10 | <10 | <10 | <10 |
| 23 | ⩾2000 | <10 | | <10 | <10 |
| 24 | ~1350 | r | | <10 | <10 |
| 25 | ⩾10 000 | <10 | <10 | <10 | r |
| 26 | ~2700 | 10 | 20 | <10 | <10 |
| 27 | ~800 | 20 r | 30 | <10 | <10 |
| 28 | ⩾3000 | <10 | 10 | <10 | <10 |
| 29 | ⩾2000 | <10 | | <10 | <10 |
| 30 | >4000 | <10 | <10 | <10 | <10 |
| 31 | 4300 | <10 | <10 | <10 | <10 |
| 32 | ⩾2000 | <10 | r | <10 | <10 |
| 33 | >1000 | <10 | 10 | <10 | 25 |
| 34 | ⩾3000 | 10 | r | <10 | r |
| 35 | ⩾4000 | 10 | r | <10 | <10 |
| 36 | ⩾3000 | <10 | r | <10 | 35 |
| ECONA-ZOLE (Vergleich) | 462.7 | <10 | <10 | <10 | <10 |

Die Abkürzungen in Tabelle I bedeuten:

St = Staphylococcus aureus haemolyticus
Str = Streptococcus faecalis
Tri = Trichophyton mentagrophytes
Asp = Aspergillus niger
r = Teilresistenz beobachtet

Beispiele 37—39

Nach der oben beschriebenen Herstellungsweise wurden folgende Verbindungen hergestellt:

37. 1-{β-[5-(p-Chlorphenylthio)-pentoxy]-2,4-dichlorphenäthyl}-imidazol in Form des Methansulfonatsalzes, F 130—130,5° C.
38. 1-{β-[5-(p-Chlorphenoxy)-pentoxy]-2,4-dichlorphenäthyl}-imidazol, ebenfalls in Form des Methansulfonatsalzes, F 138—139° C.
39. 1-{β-[5-(p-Chlorphenoxy)-pentylthio]-2,4-dichlorphenäthyl}-imidazol als Nitrat, F 88—89° C.

Diese Verbindungen sind im Verhältnis zu den Beispielen von Verbindungen (1) mit kürzerer $(CH_2)_n$-Kette etwas weniger wirksam, haben aber ebenfalls im Vergleich zum ECONA-ZOLE eine wesentlich geringere Toxizität.

Erfindungsgemäße Zubereitungen können aus den Verbindungen (1) bzw. ihren Säureadditionssalzen in an sich für antimykotische bzw. antibakterielle Mittel üblichen Weise durch Mischen, Dispergieren, Emulgieren, Lösen usw. in festen, flüssigen oder halbflüssigen Trägern, gewünschtenfalls unter Beimischung weiterer aktiver Stoffe oder/und Adjuvantien hergestellt werden. Einige Beispiele werden im folgenden angegeben:

(A) 70 Gewichtsteile Wirkstoff der Formel (1) wurden mit 3 Gewichtsteilen Maisstärke und 22 Gewichtsteilen Lactose granuliert; dann wurden weitere 3 Gewichtsteile Maisstärke und 1 Gewichtsteil Magnesiumstearat zugegeben. Das Gemisch wurde erneut granuliert und in Kapseln eingefüllt.

(B) 2 Gewichtsteile Wirkstoff der Formel (1) wurden in 10 Gewichtsteile niedermolekulares Polyäthylenglycol eingemischt und mit 88 Gewichtsteilen einer Salbenbasis vermischt.

(C) 2 Gewichtsteile Wirkstoff der Formel (1) wurden mit 9 Gewichtsteilen Maisstärke vermahlen; dann wurden 90 Gewichtsteile Talkumpulver hinzugegeben und bis zur gewünschten Bestäubungspulverfeinheit weiter vermahlen.

## Patentansprüche

1. Neue Imidazolyläthyl-oxyalkoxy-Derivate und Thio-Analoge, gekennzeichnet durch die Formel (1)

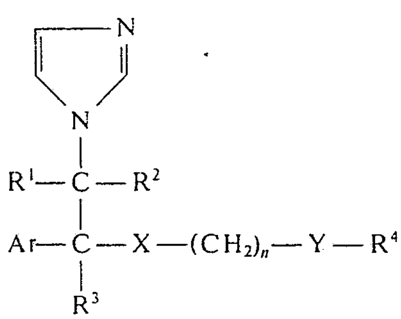

(1)

in welcher

$R^1$, $R^2$ und $R^3$ gleich oder verschieden und Wasserstoffatome oder Niederalkylgruppen sind,

$R^4$ das Wasserstoffatom, eine Niederalkylgruppe, eine $C_4$—$C_8$-Cycloalkyl-, Benzyl- oder Phenylgruppe, die gegebenenfalls mindestens einen Substituenten trägt, der ein Halogenatom oder eine Niederalkyl-, Niederalkoxy-, Niederalkylthio-, Phenyl-, Cyano-, Nitro- oder Aminogruppe ist, oder eine gegebenenfalls halogensubstituierte Pyridylgruppe bedeutet,

Ar eine Phenylgruppe bedeutet, die gegebenenfalls mindestens einen Substituenten trägt, der ein Halogenatom oder eine Niederalkyl-, Niederalkoxy- oder $C_4$—$C_8$-Cycloalkylgruppe oder eine gegebenenfalls mit einem der für $R^4$ aufgezählten Substituenten substituierte Benzylgruppe ist,

X und Y unabhängig voneinander Sauerstoff- oder Schwefelatome bedeuten und

n eine Zahl von 1 bis 5 darstellt.

sowie pharmakologisch zulässige Säureadditionssalze von Verbindungen der Formel (1).

2. Imidazolderivat nach Patentanspruch 1, dadurch gekennzeichnet, daß $R^1$ die Methylgruppe oder das Wasserstoffatom ist und $R^2$ und $R^3$ Wasserstoffatome sind, sowie pharmakologisch zulässige Säureadditionssalze hiervon.

3. Imidazolderivat nach Patentanspruch 1 oder 2, dadurch gekennzeichnet, daß $R^4$ das Wasserstoffatom, eine Niederalkylgruppe, eine gegebenenfalls halogensubstituierte Benzyl-, Cyclohexyl- oder Pyridylgruppe oder eine Phenylgruppe ist, die einen oder zwei Substituenten aus der Gruppe der Halogenatome, Niederalkylgruppen, Niederalkoxygruppen und Niederalkylthiogruppen oder eine Phenylgruppe trägt, sowie pharmakologisch zulässsige Säureadditionssalze hiervon.

4. Imidazolderivat nach Patentanspruch 3, dadurch gekennzeichnet, daß $R^4$ Methyl, Phenyl, 2-Chlorphenyl, 4-Chlorphenyl, 4-Bromphenyl, 2,4-Dichlorphenyl, 3,4-Dichlorphenyl, 4-Methylphenyl, 2-Methoxyphenyl, 4-Methoxyphenyl, 4-Methylthiophenyl, 4-Phenyl-phenyl, 3-Methyl-4-bromphenyl, Cyclohexyl, 4-Chlorbenzyl oder 5-Chlorpyrid-2-yl bedeutet, sowie pharmakologisch zulässige Säureadditionssalze hiervon.

5. Imidazolderivat nach einem der Patentansprüche 1—4, dadurch gekennzeichnet, daß n 1, 2 oder 3 ist, sowie pharmakologisch zulässige Säureadditionssalze hiervon.

6. Imidazolderivat nach einem der Patentansprüche 1—5, dadurch gekennzeichnet, daß Ar eine Phenylgruppe, die durch ein oder zwei Halogenatome, durch eine gegebenenfalls halogensubstituierte Benzylgruppe oder die Cyclohexylgruppe substituiert ist, bedeutet, sowie pharmakologisch zulässige Säureadditionssalze hiervon.

7. Imidazolderivat nach Patentanspruch 6, dadurch gekennzeichnet, daß Ar eine dihalogensubstituierte Phenylgruppe, insbesondere die 2,4-Dichlorphenylgruppe ist.

8. Pharmazeutische Zubereitungen und Präparate zur Bekämpfung von Pilzen und/oder Bakterien, insbesondere in für orale oder topische Anwendung geeigneter Form oder als sterile Injektionslösung, enthaltend mindestens eine Verbindung gemäß Patentanspruch 1 oder ein pharmakologisch zulässiges Salz einer solchen Verbindung zusammen mit einem pharmakologisch zulässigen Träger- oder Verdünnungsstoff.

9. Verfahren zur Herstellung der Imidazolderivats gemäß Formel (1) von Patentanspruch 1, dadurch gekennzeichnet, daß man die entsprechenden Alkohole bzw. Thiole der Formeln

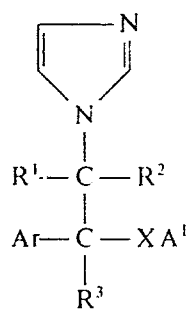

oder

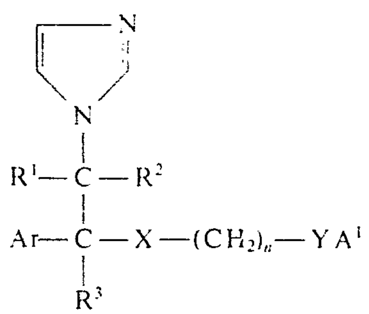

mit entsprechenden reaktiven Verbindungen der Formeln

$$A^2-(CH_2)_n-Y-R^4 \text{ bzw. } A^2-R^4$$

kondensiert, wobei $A^1$ und $A^2$ entsprechende Abgangsgruppen sind.

10. Verfahren zur Herstellung von Imidazolderivaten der Formel (1) gemäß Patentanspruch 1, dadurch gekennzeichnet, daß man die entsprechenden Imidazole der Formeln

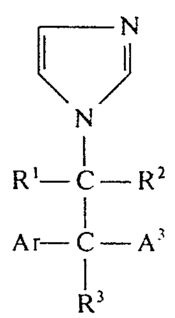

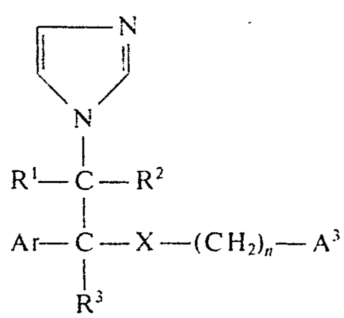

mit entsprechenden Alkoholen bzw. Thiolen der Formeln

$$A^4-X-(CH_2)_n-Y-R^4 \text{ bzw. } A^4-Y-R^4$$

kondensiert, wobei $A^3$ und $A^4$ entsprechende Abgangsgruppen sind.

## Claims

1. Novel imidazolylethyl-oxyalkoxy derivatives and thioanalogues, characterized by the formula (1)

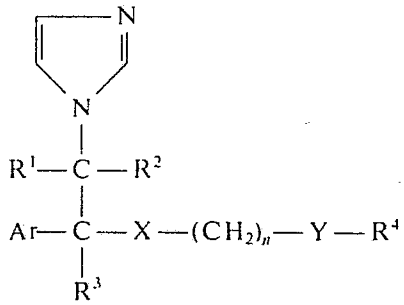

(1)

in which

| | |
|---|---|
| $R^1$, $R^2$ and $R^3$ | are the same or different and are hydrogenatoms or lower alkyl groups, |
| $R^4$ | is the hydrogen atom, a lower alkyl group, a $C_4-C_8$-cycloalkyl, benzyl or phenyl group optionally carrying at least one substituent which is a halogen atom or a lower alkyl, lower alkoxy, lower alkylthio, phenyl, cyano, nitro or amino group or signifies an optionally halogen-substituted pyridyl group, |
| Ar | signifies a phenyl group optionally carrying at least one substituent which is a halogen atom or a lower alkyl, lower alkoxy or $C_4-C_8$-cycloalkyl group or a benzyl group optionally substituted with one of the substituents enumerated for $R^4$, |
| X and Y | independently of each other signify oxygen or sulphur atoms and |
| n | is an integer of from 1 to 5, |

and pharmacologically admissible acid-addition salts of compounds of the formula (1).

2. Imidazole derivative according to claim 1, characterized in that $R^1$ is the methyl group or the hydrogen atom and $R^2$ and $R^3$ are hydrogen atoms and pharmacologically admissible acid-addition salts thereof.

3. Imidazole derivative according to claims 1 or 2, characterized in that $R^4$ is the hydrogen atom, a lower alkyl group, an optionally halogen-substituted benzyl, cyclohexyl or pyridyl group or a phenyl group having one or two substituents from the group of halogen atoms, lower alkyl groups, lower alkoxy groups and lower alkylthio groups or a phenyl group, and pharmacologically admissible acid-addition salts thereof.

4. Imidazole derivative according to claim 3, characterized in that $R^4$ is methyl, phenyl, 2-chlorophenyl, 4-chlorophenyl, 4-bromophenyl, 2,4-dichlorophenyl, 3,4-dichlorophenyl, 4-methylphenyl, 2-methoxyphenyl, 4-methoxyphenyl, 4-methylthiophenyl, 4-phenyl-phenyl, 3-methyl-4-bromophenyl, cyclohexyl, 4-chlorobenzyl or 5-chloropyrid-2-yl, and pharmacologically admissible acid-addition salts thereof.

5. Imidazole derivative according to any of claims 1—4, characterized in that n ist 1, 2 or 3 and pharmacologically admissible acid-addition salts thereof.

6. Imidazole derivative according to any of claims 1—5, characterized in that Ar is a phenyl group substituted with one or two halogen atoms or with an optionally halogen-substituted benzyl group or with the cyclohexyl group, and pharmacologically admissible acid-addition salts thereof.

7. Imidazole derivative according to claim 6, characterized in that Ar ist a dihalogen-substituted phenyl group, notably the 2,4-dichlorophenyl group.

8. Pharmaceutical compositions and preparations for control of fungi and/or bacteria, notably in a form suitable for oral or topical application or as a sterile solution for parenteral application, containing at least one compound according to claim 1 or a pharmacologically admissible salt of such a compound together with a pharmacologically acceptable carrier or diluent.

9. Process for producing the imidazole derivatives according to formula (1) of claim 1, characterized by condensing the corresponding alcohols or thiols, respectively, of the formulae

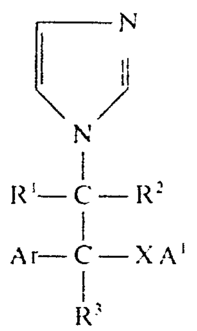

or

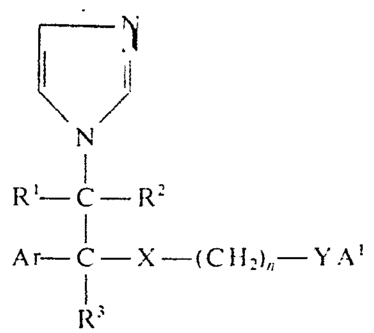

with corresponding reactive compounds of the formulae

$$A^2-(CH_2)_n-Y-R^4 \text{ or } A^2-R^4,$$

respectively wherein $A^1$ and $A^2$ are corresponding leaving groups.

10. Process for producing imidazole derivatives of the formula (1) according to claim 1, characterized by condensing the corresponding imidazoles of the formulae

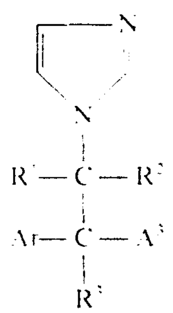

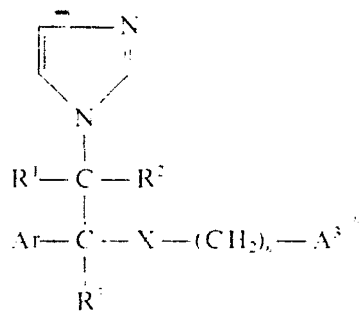

with corresponding alcohols or thiols, respectively, of the formulae

$$A^4-X-(CH_2)_n-Y-R^4 \text{ or } A^4-Y-R^4,$$

respectively, wherein $A^3$ and $A^4$ are corresponding leaving groups.

**Revendications**

1. Nouveaux composés imidazolyléthyloxyalcoxy et analogues thio de ceux-ci, caractérisés en ce qu'ils répondent à la formule (1)

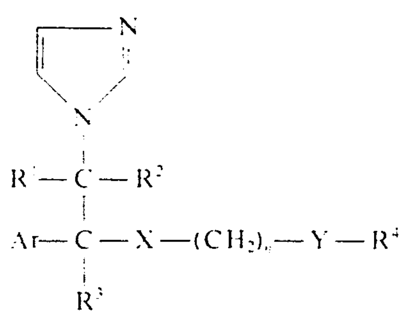

(1)

dans laquelle

$R^1$, $R^2$ et $R^3$ représentent chacun, indépendamment les uns des autres, un atome d'hydrogène ou un radical alkyle inférieur,

$R^4$ représente un atome d'hydrogène, un radical alkyle inférieur, un radical cycloalkyle en $C_4-C_8$, un radical benzyle ou phényle éventuellement porteur d'un ou plusieurs substituants pris dans l'ensemble constitué par les atomes d'halogènes et les radicaux

| | |
|---|---|
| | alkyles inférieurs, alcoxy inférieurs, alkylthio inférieurs, phényle, cyano, nitro et amino, ou encore $R^4$ représente un radical pyridyle éventuellement halogéné, |
| Ar | représente un radical phényle éventuellement porteur d'un ou plusieurs substituants choisis dans l'ensemble constitué par les atomes d'halogènes, les radicaux alkyles et alcoxy inférieurs, les radicaux cycloalkyles en $C_4-C_8$ et les radicaux benzyles éventuellement porteurs d'un des substituants énuméré pour $R^4$, |
| X et Y | représentent chacun, indépendamment l'un de l'autre, un atome d'oxygène ou de soufre et |
| n | est un entier de 1 à 5, |

et sels, acceptables du point de vue pharmacologique, formés par addition entre ces composés et des acides.

2. Composés imidazoliques selon la revendication 1, caractérisés ce que $R^1$ représente un radical méthyle ou un atome d'hydrogène et $R^2$ et $R^3$ représentent des atomes d'hydrogène, ainsi que leurs sels d'addition d'acides acceptables du point de vue pharmacologique.

3. Composés imidazoliques selon l'une des revendications 1 et 2, caractérisés en ce que $R^4$ représente un atome d'hydrogène, un radical alkyle inférieur, un radical benzyle, cyclohexyle ou pyridyle éventuellement halogéné, ou un radical phényle porteur d'un ou deux substituants pris dans l'ensemble constitué par les atomes d'halogènes, les radicaux alkyles inférieurs, les radicaux alcoxy inférieurs, les radicaux alkylthio inférieurs et le radical phényle, ainsi que leurs sels d'addition d'acides acceptables du point de vue pharmacologique.

4. Composés imidazoliques selon la revendication 3, caractérisés en ce que $R^4$ représente un radical méthyle, phényle, chloro-2 phényle, chloro-4 phényle, bromo-4 phényle, dichloro-2,4 phényle, dichloro-3,4 phényle, méthyl-4 phényle, méthoxy-2 phényle, méthoxy-4 phényle, méthylthio-4 phényle, phényl-4 phényle, méthyl-3 bromo-4 phényle, cyclohexyle, chloro-4 benzyle ou chloro-5 pyridyle-2, ainsi que leurs sels d'addition acceptables du point de vue pharmacologique.

5. Composés imidazoliques selon l'une quelconque des revendications 1 à 4, caractérisés en ce que n est égal à 1, à 2 ou à 3, ainsi que leurs sels d'addition acceptables du point de vue pharmacologique.

6. Composés imidazoliques selon l'une quelconque des revendications 1 à 5, caractérisés en ce que Ar représente un radical phényle porteur d'un ou deux atomes d'halogènes, d'un radical benzyle éventuellement halogéné ou d'un radical cyclohexyle, ainsi que leurs sels d'addition d'acides acceptables du point de vue pharmacologique.

7. Composés imidazoliques selon la revendication 6, caractérisés en ce que Ar représente un radical phényle dihalogéné, plus particulièrement un radical dichloro-2,4 phényle.

8. Compositions pharmaceutiques et préparations pour combattre des mycètes et/ou des bactéries, plus spécialement sous une forme appropriée pour l'application orale ou locale ou sous la forme d'une solution stérile injectable, caractérisées en ce qu'elles contiennent au moins un composé selon la revendication 1 ou un sel, acceptable du point de vue pharmacologique, d'un tel composé, avec un support ou diluant acceptable du point de vue pharmacologique.

9. Procédé de préparation des composés imidazoliques de formule (1) selon la revendication 1, procédé caractérisé en ce qu'on condense les alcools ou thiols correspondants qui répondent à l'une des formules:

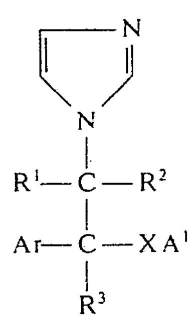

et

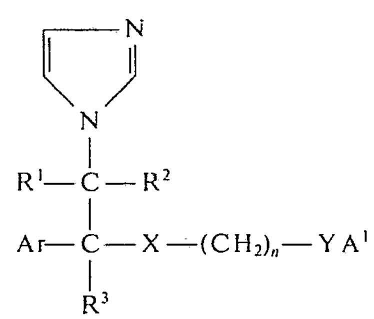

avec des composés réactifs correspondants qui répondent respectivement à la formule

$A^2-(CH_2)_n-Y-R^4$ ou à la formule $A^2-R^4$

formules dans lesquelles $A^1$ et $A^2$ désignent des groupes éliminables qui se correspondent.

10. Procédé de préparation de composés imidazoliques de formule (1) selon la revendication 1, procédé caractérisé en ce qu'on condense les imidazoles correspondants qui répondent à l'une des formules:

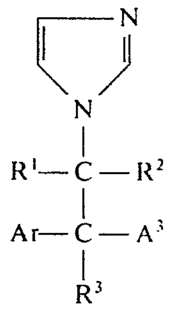

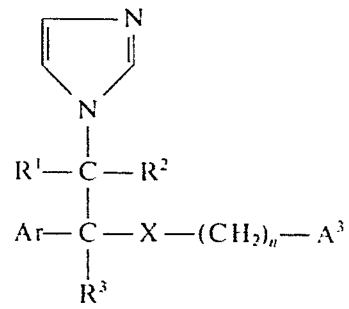

avec des alcools ou thiols correspondants qui répondent respectivement à la formule

$$A^4-X-(CH_2)_n-Y-R^4 \text{ ou à la formule } A^4-Y-R^4$$

formules dans lesquelles $A^3$ et $A^4$ représentent des groupes éliminables qui se correspondent.